# EUROPÄISCHE PATENTSCHRIFT

(11) **EP 0 622 058 B1**
(45) Veröffentlichungstag und Bekanntmachung des Hinweises auf die Patenterteilung: **02.06.1999**
(21) Anmeldenummer: 94850068.1
(22) Anmeldetag: 26.04.1994
(51) Int. Cl.: A61F 2/00, A61F 2/30, A61C 8/00, A61B 17/58

(54) **Implantierbares Verankerungsorgan zur Aufnahme von Prothesen, künstlichen Gelenkteilen u.dgl.**
Implantable anchoring organ for receiving prostheses, artificial joint parts and the like
Organe d'ancrage implantable pour la réception de prothèses de parties d'articulation artificielles et d'objets similaires

(30) Priorität: 27.04.1993 SE 9301407
(43) Veröffentlichungstag der Anmeldung: 02.11.1994
(73) Patentinhaber: MEDEVELOP AKTIEBOLAG, S-402 29 Göteborg (SE)
(72) Erfinder: Branemark, Per-Ingvar, S-431 69 Mölndal (SE)
(74) Vertreter: Karlsson, Leif Karl Gunnar

(56) Entgegenhaltungen:
- EP-A- 0 179 736
- EP-A- 0 230 856
- WO-A-91/18556
- DE-A- 2 747 312

## Beschreibung

Die vorliegende Erfindung betrifft ein zur Implantation in Geweben, vorzugsweise Knochengeweben, vorgesehenes, im wesentlichen rotationssymmetrisch ausgebildetes und aus einem gewebefreundlichem Material bestehendes Verankerungsorgan zur Halterung von Prothesen, Amputationsprothesen, künstlichen Gelenkteilen u.dgl, mit einem wenigstens teilweise entlang seiner Manteloberfläche angeordneten, in Richtung zu seinem Einführungsende verlaufenden Aussengewinde.

Implantierbare Verankerungsorgane der eingangs erwähnten Art werden beispielsweise seit vielen Jahren sehr erfolgreich im Dentalbereich zum Verankern einzelner künstlicher Zähne und auch Zahnbrücken verwendet. Derartige Verankerungsorgane oder Fixturen sind beispielsweise in US-A- 5 064 425 beschrieben und zum Einschrauben in einer im Knochengewebe vorgefertigten Bohrung vorgesehen; nach dem Einschrauben in diese Bohrung wird der Fixtur die Möglichkeit gegeben, normalerweise während längerer Zeit (etwa 4 - 6 Monate) belastungslos in der Bohrung mit dem umgebenden Gewebe verknöchernd festzuwachsen. Im Anschluss daran wird der obere Teil der Fixtur zwecks Anschluss der erwähnten Zähne oder Zahnbrücken über geeignete Abstandselemente oftmals freigelegt.

Derartige Verankerungsorgane oder Fixturen bestehen grundsätzlich aus einem mit einem Aussengewinde versehenen, schraubenförmigen Titankörper, wobei die dem Gewebe zugewandte Oberfläche der Körpers und natürlich auch das Gewinde eine besondere Oberflächenstruktur zur Erzielung eines optimalen biologischen Zusammenwirkens, einer s.g. Osseointegration, mit dem umgebenden Gewebe aufweist. Diese Oberflächenstruktur umfasst eine grosse Anzahl von in der Oberfläche angeordneten Mikro- und Makrodellen, die Lagerungs-und Befestigungspunkte für die umgebenden Gewebezellen und deren Zellenausläufern darstellen (US-A- 4 550 891 und EP 0 338 576). Zusammen mit einer zu diesem Zweck entwickelten schonenden Operationstechnik wird das notwendige Zusammenspiel zwischen der mit Mikrodellen und etwaigen Makrodellen versehenen Implantatoberfläche und den Gewebezellen bzw. Zellenausläufern erzielt, wodurch die angestrebte biologische Verankerung sichergestellt wird.

Weiter wird in der EP-A- 230 856 ein Verankerungselement mit Einkerbungen auf einer gewindeverssehenen Partie des Elements beschrieben. Diese Einkerbungen sind bedeutend tiefer als das Gewinde.

Eine umfassende klinische Langzeitbeobachtung solcher Implantate zeigt, dass die Fixturen bezüglich ihrer axialem Belastung ausgezeichnete Verankerungseigenschaften aufweisen.

Im Zusammenhang mit der neuzeitlich vorgeschlagenen Anwendung derartiger Verankerungsorgane zur Aufnahme von Prothesenteilen, wie Fingergelenke, Hüftgelenke, Fussgelenke, Handgelenke u.s.w., hat sich jedoch erwiesen, dass man aufgrund des Vorliegens anderer als rein axialer Belastungsverhältnisse auch die Drehstabilität der Fixturen beachten muss. Dies ist notwendig, um die Gefahr einer Lockerung der Fixtur bei wiederholter Drehbelastung, die jeweils der Fixtur eine seitliche Lageveränderung unterwirft, zu vermeiden und, auch im Falle eines Festwachsens der Fixtur in ihrer neuen Lage, durch Bildung von Bindegewebe nach und nach eine Verschlechterung des Verankerungsvermögens der Fixtur im Gewebe ergibt.

Gegenstand vorliegender Erfindung ist eine weitmöglichste Verminderung der oben genannten Probleme und Schwierigkeiten und eine Weiterausbildung der Fixtur derart, dass man unter Beibehaltung der früheren axialen Belastungkapazität die Drehstabilität der Fixtur wesentlich erhöht und dadurch die Gefahr einer Lockerung des Verankerungsorgans so weit wie möglich ausschaltet.

Bei einem eingangs beschriebenen Verankerungsorgan wird diese Aufgabe hauptsächlich dadurch gelöst, dass das Verankerungsorgan die besonderen Merkmale, die in dem kennzeichnenden Teil des Palentanspruchs / angegeben sind, aufzeigt.

Nach einer bevorzugten Ausführungsform der Erfindung werden diese Einkerbungen von wenigstens einer im Aussengewinde des Verankerungsorgans vorgesehenen, spiralförmig verlaufenden Nut gebildet, wobei die Nut eine bedeutend grössere Steigung als die des Aussengewindes aufweist.

Zweckmässig ist die Steigung der spiralförmigen Nut mindestens dreimal so gross wie die Steigung des Aussengewindes.

Hierbei können die Einkerbungen zweckmässig in Abständen voneinander längs einer gedachten spiralförmigen Linie angeordnet sein.

Im Rahmen der Erfindung können auch zwei oder meherere in Abständen voneinander angeordnete spiralförmige Nuten vorgesehen sein und weiters können diese Nuten sich auch längs des gesamten Aussengewindes erstrecken.

Die Einkerbungen können auch von hauptsächlich axial verlaufenden Nuten gebildet werden. Dabei können diese axialen Nuten sich zweckmässig über wenigstens fünf Windungen des Aussengewindes erstrecken. Solche axialen Nuten können auch in ihrer Lage gegenseitig verschoben sein.

Zweckmässig weisen die Nuten einen keilförmigen Querschnitt auf. Die Gewindespitzen sind abgerundet und die Gewindesohlen sind zweckmässig weniger tier als in Normalgewinden.

Die Erfindung wird nachstehend an Hand einiger in beiliegenden Zeichnungen gezeigten Ausführungsberspielen näher beschreiben, wobei
- Fig. 1: ein konventionelles Verankerungsorgan in Seitenansicht zeigt,
- Fig. 2: eine Draufsicht auf den Gegenstand nach Fig. 1 darstellt,
- Fig. 3: einen Schnitt durch das Gewinde des Gegenstandes nach Fig 1 darstellt,
- Fig. 4: ein erfindungsgemäss vorgeschlagenes Verankerungsorgan nach einer ersten Ausführungsform in perspektivischer Ansicht zeigt,
- Fig. 5: eine perspektivische Ansicht eines Teils des erfindungsgemäss vorgeschlagenen Verankerungsorgans nach einer zweiten Ausführungsform darstellt und
- Fig. 6: die Manteloberfläche nach Fig. 5 in vergrössertem Masstab zeigt.

In den Zeichnungen wird ein zur Implantation bestimmtes Verankerungsorgan allgemein mit 1 bezeichnet.

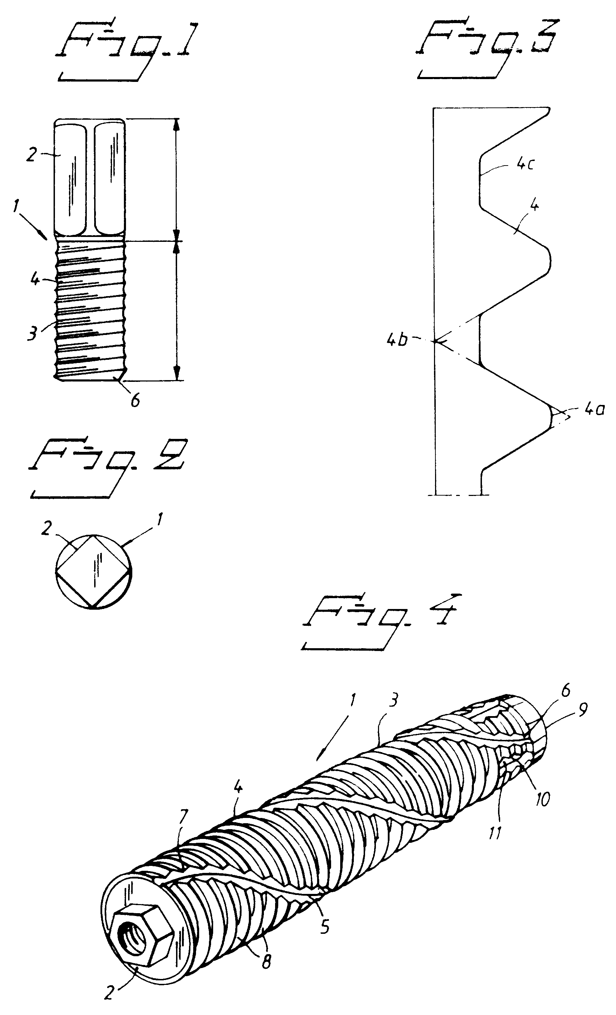

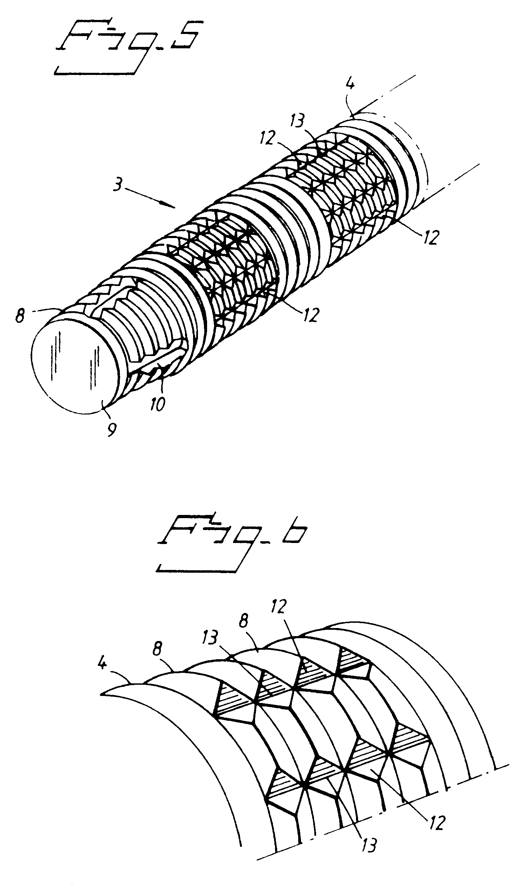

In Fig. 1 - 3 wird ein konventionelles Verankerungsorgan 1 gezeigt. Ein solches Verankerungsorgan besteht zweckmässig aus einem gewebefreundlichen Material, vorzugsweise Titan, und umfasst ein oberes Anschlussteil 2 für den Anschluss von Prothesenteilen, künstlichen Gelenkteilen, u.s.w. und ein unteres Verankerungsteil 3, das entlang seiner Mantelfläche mit einem auf das untere Ende des Organes 1, d.h. auf das Einführungsende 6 zu verlaufenden Aussengewinde 4 versehen ist. In der hier gezeigten Ausbildung ist das Gewinde 4 ein Rechtsgewinde. Eine zweckmässige Ausbildung eines derartigen Aussengewindes 4 ist in Fig. 3 veranschaulicht. Hieraus ist ersichtlich, dass die initial spitze Gewindespitze abgerundet wurde, während die initial spitzenförmige Ausbildung der Gewindesohle 4b durch eine Abflachung 4c ersetzt wurde.

Zwecks optimaler biologischer Verankerung im Gewebe sind wenigstens die dem Gewebe zugeehrten Bereiche des Verankerungsorgans 1 mit einer besonderen Oberflächenstruktur ausgebildet, die dicht aneinander liegende Mikro- und etwaige Makrodellen aufweist. Der Fachmann versteht unter dem Ausdruck Mikrodellen Vertiefungen in der Implantatfläche innerhalb der Grössenordning von 10 - 10.000 nm, vorzugsweise 10 - 300 nm, und unter dem Ausdruck Makrodellen Vertiefungen in der Oberfläche im Grössenbereich von einigen µm und mehr. Verschiedene Verfahren zur Herstellung von Mikrodellen bzw. Makrodellen sind bekannt und beispielsweise in US-A- 4 330 891 und EP 0 338 576 beschrieben.

Derartige Mikrodellen sind zur Bildung von Lagerungs- und Befestigungspunkten für die Gewebezellen bzw. deren Zellausläufern vorgesehen und sind, zusammen mit einer schonenden Operationstechnik, die notwendige Voraussetzung für die angestrebte Osseointegration, d.h. Verknöcherung, zwischen Implantatoberfläche und umgebendem Gewebe. Umfangreiche klinische Studien haben gezeigt, dass eine solche Oberflächenstruktur zu einem unübertroffenen Verankerungsresultate zwischen Implantatoberfläche und umgebendem Gewebe führt.

In Fig. 4 - 6 werden zwei verschiedene Ausführungsformen eines erfindungsgemäss vorgeschlagenen Verankerungsorganes gezeigt. Bei der in Fig. 4 gezeigten Ausbildung ist eine spiralenförmig verlaufende Nut 5 im Aussengewinde 4 angeordnet, wobei die Nut 5 sich vom oberen Ende 2 des Implantes in Richtung zu dessen unteres Einführungsende 6 erstreckt. Hierbei entspricht die Tiefe der spiralenförmigen Nut 5 hauptsächlich der Tiefe des Aussengewindes 4 und die Nut kann zweckmässig keilförmig ausgebildet sein. Wie aus Fig. 4 hervorgeht, verläuft die spiralenförmige Nut 5 mit bedeutend grösserer Steigung als die des Aussengewindes 4 und bildet somit eine Einkerbung 7 in jeder Windung 8 des Aussengewindes 4. Um das Einschrauben des Organes in die vorpräparierte Bohrung nicht zu beeinflussen, soll die spiralenförmige Nut in gleicher Richtung wie das Aussengewinde 4 laufen.

Natürlich sind im Rahmen der Erfindung viele Varianten der in Fig. 4 gezeigten Ausführung möglich. Demnach ist es möglich, die spiralenförmige Nut 5 nur zonenweise entlang des Aussengewindes vorzusehen, wobei sich diese Nut hierbei beispielsweise jeweils über wenigstens fünf Windungen des Aussengewindes erstreckt. Weiters können mehrere in Abständen voneinander verlaufende Nuten 5 vorgesehen sein oder auch können die Einkerbungen in Abständen voneinander entlang einer spiralenförmigen Linie angeordnet werden.

Im übrigen ist das in Fig. 4 gezeigte Verankerungsorgan von sich aus gewindeschneidend, d. h. weist beispielsweise die im U.S. 5 064 425 beschriebene Ausbildung auf, mit einem geschlossenen Boden 9 und einer Anzahl in kurzem Abstand oberhalb des genannten geschlossenen Bodens angeordneten, axial verlaufenden Schlitzen 10, die mit im Inneren des Organes 1 vorhandenen Höhlungen 11 zum Sammeln von beim Einschrauben des Verankerungsorgans 1 in eine im Gewebe vorpräparierte Bohrung anfallende "Gewebespäne" zusammenwirken. Hierbei sind die Schlitze 10 mit geeigneten Schneidkanten ausgebildet.

Selbstverständlich ist die erfindungsgemäss vorgeschlagene Anordnung einer spiralenförmig verlaufenden Nut entlang des Aussengewindes bezüglich ihrer Verwendung nicht auf die in Fig. 4 und Fig. 5 gezeigten besonderen Verankerungsorgane nach US-A- 5 064 425 beschränkt, sondern die in Fig. 4 gezeigte Nut und auch die in Fig. 5 gezeigten axialen Nuten können selbstverständlich auch für Verankerungsorgane ohne geschlossenen Boden und ohne selbstschneidende Eigenschaften verwendet werden.

In Fig. 5 und 6 wird eine alternative Ausbildung der erfindungsgemäss vorgeschlagenen, die Drehstabilität erhöhenden Einkerbungen 7 gezeigt und hierbei sind anstatt der in Fig. 4 gezeigten spiralförmig verlaufenden Nut 5 axial verlaufende Nuten 13 vorgesehen, die die genannten Einkerbungen im Aussengewinde 4 bilden. Hierbei weisen die axialen Nuten 13 zweckmässig auch die gleiche Tiefe wie die des Aussengewindes 4 auf und können dicht aneinander liegen oder in grösseren Abständen voneinander vorgesehen sein. Diese axialen Nuten 13 können auch in ihrer Lage versetzt angeordnet sein und sollten zweckmässig eine solche Länge aufweisen, dass sie wenigstens Einkerbungen 12 in einer Anzahl, zweckmässig wenigstens drei, nebeneinander liegenden Windungen 8 des Aussengewindes 4 bilden.

Wie in Fig. 5 gezeigt, können solche axialen Nuten 13 zweckmässig zonenweise entlang des Aussengewindes 4 vorgesehen sein und hierbei wenigstens in den Zonen des Verankerungsorgans, die in lasttragendem Kontakt mit dem umgebenden Knochengewebe kommen. Die axialen Nuten 13 können zweckmässig auch keilförmig ausgebildet sein und aus Fig. 6 geht deutlich hervor, wie diese axialen Nuten 13 die erfindungsgemäss angestrebten Einkerbungen 12 im Aussengewinde 4 ergeben.

Versuche haben gezeigt, dass erfindungsgemäss vorgeschlagene, mit drehstabilisierenden Einkerbungen versehene Verankerungsorgane eine, im Vergleich zu bisher bekannten Verankerungsorganen, bedeutende Verbesserung der Drehstabilität ergeben. Es konnte somit deutlich festgestellt werden, dass man durch ein Einwachsen des umgebenden Zellmateriales in die genannte drehstabilisierenden Nut die Gefahr eines "Wiederaufschraubens" der Fixtur bei etwaigen Drehbelastungen eliminiert. Hierüberhinaus wurde festgestellt, dass die erfindungsgemäss vorgeschlagenen Einkerbungen weder auf die Funktion des Aussengewindes 4 beim Einschrauben des Verankerungsorgans in die im Gewebe vorpräparierte Bohrung einwirken noch die durch das Aussengewinde selbst erzielte Stabilität bei axialer Belastung beeinflussen.

Die Erfindung ist jedoch nicht auf den oben beschriebenen und in den Zeichnungen gezeigten Erfindungsgedanken beschränkt, sondern kann im Rahmen der nachstehenden Patentansprüche vielfach abgewandelt werden. Somit kann der Gewindetyp des Aussengewindes 4 hauptsächlich mit der in Fig. 3 gezeigten, schon früher bekannten Ausführung übereinstimmen. Weiters können solche Verankerungsorgane auch für Hautdurchtritte und andere Zwecke verwendet werden, beispielsweise zur Aufnahme von elektrischen Leitungen. Zweckmässig kann ein erfindungsgemäss vorgeschlagenes Verankerungsorgan auch zum Halten von Hüftgelenken verwendet werden und hierbei eines solche Länge aufweisen, dass es mit Sicherheit im Gewebe entlang des Knochens verankert werden kann. Gerade bei Hüftgelenkprothesen besteht ein grosser Bedarf an drehstabilisierten Fixturen, da man, wie bekannt, grosse Schwierigkeiten mit derzeit bekannten Verankerungen hat, die sehr oft schon nach relativ kurzer Anwendungsdauer eine Tendenz zur Lockerung zeigen.

## Patentansprüche

1. Zur Implantation in Geweben, vorzugsweise Knochengeweben, vorgesehenes, im wesentlichen rotationssymmetrisch ausgebildetes und aus einem gewebefreundlichen Material bestehendes Verankerungsorgan (1) zur Halterung von Prothesen, Amputationsprothesen, künstlichen Gelenkteilen u.dgl, mit einem wenigstens teilweise entlang seiner Manteloberfläche angeordneten, in Richtung zu seinem Einführungsende verlaufenden Aussengewinde (4), welcher gewindeversehene Teil wenigstens annähernd zylinderförmig ist, wobei die gewindeversehene Manteloberfläche wenigstens zonenweise mit die Drehstabilität des Organes erhöhenden, im Aussengewinde (4) angeordneten Einkerbungen (7, 12) ausgebildet ist, wobei sich die genannte Zone über mehrere Windungen erstreckt, wobei in jeder in der genannten Zone vorhandenen Windung (8) des Aussengewindes (4) wenigstens eine Einkerbung (7, 12) angeordnet ist, dadurch gekennzeichnet dass die Einkerbungen (7, 12) eine Tiefe aufweisen, die der Tiefe des Aussengewindes (4) entspricht, und dass die Gewindespitzen des Aussengewindes (4) abgerundet sind.

2. Verankerungsorgan nach Anspruch 1, dadurch gekennzeichnet, dass die Einkerbungen (7) von wenigstens einer im Aussengewinde (4) des Verankerungsorgans (1) vorgesehenen, spiralförmig verlaufenden Nut (5) gebildet werden und dass die Nut (5) eine bedeutend grössere Steigung als die des Aussengewindes (4) aufweist.

3. Verankerungsorgan nach Anspruch 1 oder 2, dadurch gekennzeichnet, dass die Steigung der spiralförmigen Nut (5) wenigstens dreimal so gross ist wie die Steigung des Aussengwindes (4).

4. Verankerungsorgan nach Anspruch 2 oder 3, dadurch **gekennzeichnet,** dass die Einkerbungen (7) im Aussengewinde in Abständen voneinander entlang einer spiralförmigen Linie vorgesehen sind.

5. Verankerungsorgan nach Anspruch 3 oder 4, dadurch **gekennzeichnet**, dass zwei oder mehrere in Abständen voneinander vorgesehene spiralförmige Nuten (5) vorgesehen sind.

6. Verankerungsorgan nach Anspruch 2 bis 5, dadurch gekennzeichnet, dass die spiralförmigen Nuten (5) sich entlang des gesamten Aussengewindes (4) erstrecken.

7. Verankerungsorgan nach Anspruch 1, dadurch gekennzeichnet, dass die Einkerbungen (12) im Aussengewinde (4) aus hauptsächlich axial verlaufenden Nuten (13) gebildet werden.

8. Verankerungsorgan nach Anspruch 7, dadurch gekennzeichnet , dass die genannten axialen Nuten (13) sich über wenigstens fünf Windungen (8) des Aussengewindes (4) erstrecken.

9. Verankerungsorgan nach Anspruch 6 bis 8, dadurch gekennzeichnet, dass die Einkerbungen (12) von wenigstens zwei im Abstand voneinander vorgesehenen axialen Nuten (13) gebildet werden.

10. Verankerungsorgan nach Anspruch 9, dadurch **gekennzeichnet,** dass die genannten, in Abstand voneinander vorgesehenen axialen Nuten (13) in ihrer Lage versetzt angeordnet sind.

11. Verankerungsorgan nach Anspruch 1 bis 9, dadurch gekennzeichnet , dass die die Einkerbungen (7, 11) bildenden Nuten (5, 13) einen keilförmigen Querschnitt aufweisen.

12. Verankerungsorgan nach Anspruch 1, dadurch **gekennzeichnet**, dass das Verankerungsorgan (1) aus Titan besteht oder eine Oberflächenbeschichtung aus Titan aufweist.

## Claims

1. An anchoring element (1) intended for implantation in tissues, preferably bone tissues, which is essentially rotationally symmetrical in form and consists of a tissue-compatible material, for retaining prostheses, amputation prostheses, artificial joint elements and suchlike, with an external screw thread (4), running towards its insertion end, disposed along at least a portion of its circumferential surface, which threaded portion is at least approximately cylindrical in form, the threaded circumferential surface being at least zonally fashioned with indentations (7, 12), disposed in the external screw thread (4), which increase the torsional stability of the element, the said zone extending over several turns and at least one indentation (7, 12) being located in each turn (8) of the external screw thread (4) present in said zone, characterised in that the indentations (7, 12) are of a depth which corresponds to the depth of the external screw thread (4) and that the thread crests of the external screw thread (4) are rounded.

2. An anchoring element according to Claim 1, characterised in that the indentations (7) are formed by at least one helical groove (5) provided in the external screw thread (4) of the anchoring organ (1) and that the groove (5) has a pitch which is significantly greater than that of the external screw thread (4).

3. An anchoring element according to Claim 1 or 2, characterised in that the pitch of the helical groove (5) is at least three times as great as the pitch of the external screw thread (4).

4. An anchoring element according to Claim 2 or 3, characterised in that the indentations (7) in the external screw thread are provided, spatially separated from one another, along a helical line.

5. An anchoring element according to Claim 3 or 4, characterised in that two or more spatially separated helical grooves (5) are provided.

6. An anchoring element according to Claims 2 to 5, characterised in that the helical grooves (5) extend along the entire external screw thread (4).

7. An anchoring element according to Claim 1, characterised in that the indentations (12) in the external screw thread (4) are formed from essentially axial grooves (13).

8. An anchoring element according to Claim 7, characterised in that the said axial grooves (13) extend over at least five turns (8) of the external screw thread (4).

9. An anchoring element according to Claims 6 to 8, characterised in that the indentations are formed by at least two spatially separated axial grooves (13).

10. An anchoring element according to Claim 9, characterised in that the said spatially separated axial grooves (13) are offset in respect of their position.

11. An anchoring element according to Claim 1 to 9, characterised in that the grooves (5, 13) forming the indentations (7, 12) have a wedge-shaped cross section.

12. An anchoring element according to Claim 1, characterised in that the anchoring element (1) consists of titanium or has a surface coating of titanium.

## Revendications

1. Organe d'ancrage (1) prévu pour l'implantation dans des tissus, de préférence dans des tissus osseux, constitué de façon essentiellement symétrique en rotation et se composant d'un matériau histocompatible, servant à fixer des prothèses, des prothèses d'amputation, des pièces articulées artificielles et des pièces analogues, comprenant un filetage extérieur (4) s'étendant en direction de son extrémité d'entrée, disposé au moins partiellement le long de sa surface latérale, laquelle pièce filetée est de forme au moins presque cylindrique, où la surface latérale filetée est constituée, au moins par zones, d'entailles (7, 12) disposées dans le filetage extérieur (4) et augmentant la stabilité de rotation de l'organe, où ladite zone s'étend sur plusieurs spires et où au moins une entaille (7, 12) est disposée dans chaque spire (8) du filetage extérieur (4) présente dans ladite zone,
caractérisé en ce que les entailles (7, 12) ont une profondeur qui correspond à la profondeur du filetage extérieur (4) et en ce que les sommets du filet du filetage extérieur (4) sont arrondis.

2. Organe d'ancrage selon la revendication 1, caractérisé en ce que les entailles (7) sont formées au moins par une rainure (5) prévue dans le filetage extérieur (4) de l'organe d'ancrage (1) et s'étendant en forme de spirale, et en ce que la rainure (5) présente un pas beaucoup plus grand que celui du filetage extérieur (4).

3. Organe d'ancrage selon la revendication 1 ou 2, caractérisé en ce que le pas de la rainure (5) en forme de spirale est égal à au moins trois fois le pas du filetage extérieur (4).

4. Organe d'ancrage selon la revendication 2 ou 3, caractérisé en ce que les entailles (7) dans le filetage extérieur sont prévues en étant espacées les unes des autres par des intervalles le long d'une ligne en forme de spirale.

5. Organe d'ancrage selon la revendication 3 ou 4, caractérisé en ce que deux rainures (5), ou plus, en forme de spirale, sont prévues en étant espacées les unes des autres par des intervalles.

6. Organe d'ancrage selon l'une quelconque des revendications 2 à 5, caractérisé en ce que les rainures (5) en forme de spirale s'étendent sur la totalité du filetage extérieur (4).

7. Organe d'ancrage selon la revendication 1, caractérisé en ce que les entailles (12) dans le filetage extérieur (4) sont constituées principalement de rainures (13) s'étendant axialement.

8. Organe d'ancrage selon la revendication 7, caractérisé en ce que lesdites rainures axiales (13) s'étendent sur au moins cinq spires (8) du filetage extérieur (4).

9. Organe d'ancrage selon l'une quelconque des revendications 6 à 8, caractérisé en ce que les entailles (12) sont constituées d'au moins deux rainures axiales (13) prévues en étant espacées l'une de l'autre par un intervalle.

10. Organe d'ancrage selon la revendication 9, caractérisé en ce que lesdites rainures axiales (13), prévues en étant espacées l'une de l'autre par un intervalle, sont disposées en étant décalées dans leur position.

11. Organe d'ancrage selon l'une quelconque des revendications 1 à 9, caractérisé en ce que les rainures (5, 13) formant les entailles (7, 11) ont une section cunéiforme.

12. Organe d'ancrage selon la revendication 1, caractérisé en ce que l'organe d'ancrage (1) est en titane ou qu'il présente une couche de surface en titane.
